# EUROPEAN PATENT APPLICATION

(11) **EP 4 691 467 A1**
(43) Date of publication of application: **11.02.2026**
(21) Application number: 23935156.2
(22) Date of filing: 21.12.2023
(51) Int. Cl.: A61K 31/513, A61P 29/00, A61P 19/06, A61P 19/02

(54) **USE OF INHIBITOR SPECIFICALLY TARGETING NLRP3 INFLAMMASOME**

(30) Priority: 28.04.2023 CN 202310506485
(71) Applicant: Sichuan Jianlin Pharmaceutical Co., Ltd, Chengdu, Sichuan 610100 (CN)
(72) Inventor: ZHANG, Xing, Xi'an, Shaanxi 710032 (CN); LIU, Xuyun, Xi'an, Shaanxi 710032 (CN); QIN, Xiangyang, Xi'an, Shaanxi 710032 (CN)
(74) Representative: Gille Hrabal Partnerschaftsgesellschaft mbB Patentanwälte
(86) International application number: PCT/CN2023/140742
(87) International publication number: WO 2024/221990

(57) **Abstract**

Use of an inhibitor specifically targeting NLRP3 inflammasome. 5-FUMCL inhibits IL-13 and IL-6 secretion in an LPS-induced acute systemic inflammation model, and has significant inhibitory effect on inflammatory cytokines IL-1B, IL-6 and IL-18 of acute peritonitis and gouty arthritis induced by the injection of MSU into abdominal cavity and knee joint cavity, wherein the number of neutrophils in the abdominal cavity can be significantly reduced and the swelling degree of the knee joint can be improved. The corresponding anti-inflammatory effect is not shown in a model in which NLRP3 molecule is knocked out. Experimental results show that 5-FUMCL is an inhibitor specifically targeting NLRP3 inflammasome, and can be used as an active ingredient in the preparation of a drug for preventing or treating NLRP3 inflammasome-related diseases, and particularly has a significant treatment effect on inflammation-related diseases such as acute systemic inflammation, acute peritonitis, and gouty arthritis.

## Description

### TECHNICAL FIELD

The present disclosure pertains to the technical field of pharmaceuticals, relates to an inhibitor specifically targeting NLRP3 inflammasome, and particularly relates to anti-inflammatory use of 5-FUMCL.

### BACKGROUND

Inflammation is a self-protective response generated by organisms when they are attacked by external forces or under environmental stress, and it plays a very important role in maintaining the health of organisms. However, long-term uncontrollable inflammation in organisms can cause their damage, thereby leading to the development of diseases. After the concept of inflammasome was first proposed by Tschopp's laboratory in 2002, research on the NLRP3 inflammasome has become a hot field. NLRP3 comprises three domains, which are an N-terminal pyrin domain (PYD), an ATP-catalytic nucleotide-binding oligomerization domain (NACHT), and a C-terminal leucine-rich repeat (LRR). A host induces the activation of nuclear factor-κB (NF-κB) by recognizing pathogen-associated molecular patterns (PAMPs) and damage-associated molecular patterns (DAMPs), thereby triggering the activation of NLRP3. The pyrin domain (PYD) of the activated NLRP3 binds to the PYD domain of ASC and assembles with the ASC helix to form ASC specks. Subsequently, the Caspase recruitment domain (CARD) of ASC interacts with and binds to the CARD of pro-caspase-1, and generates caspase-1 through the proteolytic cleavage of procaspase-1 in the form of an oligomer, which in turn produces various biological effects by mediating the processing, maturation, and secretion of interleukins IL-1β and IL-18, regulating the expression of inflammation-related genes, and the like, thereby causing inflammation. Caspase-1 also cleaves gasdermin D (GSDMD) into N-terminal gasdermin-D, thereby producing membrane pores and causing pyroptosis. The overactivation of the NLRP3 inflammasome is associated with a variety of diseases, including cryopyrin-associated periodic syndrome (CAPS), familial cold autoinflammatory syndrome (FCAS), Muckle-Wells syndrome (MWS), inflammatory bowel disease (IBD), type II diabetes, obesity, non-alcoholic steatohepatitis, aging, atherosclerosis, gout, renal dysfunction, Alzheimer's disease (AD), Parkinson syndrome, sepsis, endotoxemia, peritonitis, war wound, cardiovascular stress, and myocardial hypertrophy, and is associated with a variety of cancers, such as colon cancer, breast cancer, melanoma, hepatitis C virus-associated hepatocellular carcinoma, gastrointestinal cancer, and the like.

The NLRP3 inflammasome is located upstream of inflammatory cytokines, is an important initiating factor for inflammation-related diseases, and has become an important target for the development of novel and specific anti-inflammatory drugs. However, existing clinical therapeutic drugs for NLRP3 inflammasome-related diseases mainly act by indirectly inhibiting NLRP3 inflammasome components or related signal events, such as IL-18 receptor antagonists, IL-1β neutralizing antibodies, IL-18 receptor blockers, and the like. Although these drugs have shown relatively good efficacy in clinical use, the mechanism of action or the exact targets of the drugs have not been fully elucidated. It is noted that not only IL-18 and IL-1β are produced when the NLRP3 inflammasome is activated, but also these inflammatory cytokines may be produced by other inflammasomes such as NLRC4 and AIM2 after activation, or may be released through non-inflammasome-mediated pathways, and in addition, HMGB1 and the like released during pyroptosis may also involve in the development and progression of NLRP3 inflammasome-related inflammatory diseases. Therefore, simply inhibiting these downstream inflammatory cytokines not only risks off-target effects, but also may cause side effects such as immunosuppressive effects and the like. The research and development of small-molecule drugs targeting the NLRP3 inflammasome is severely lagging, and several biotechnology and pharmaceutical companies are mainly focusing on the research and development of NLRP3 inhibitors. However, at present, there are still no anti-inflammatory drugs that directly target the NLRP3 inflammasome for clinical use.

In the process of exploring and developing anti-inflammatory drugs that directly target the NLRP3 inflammasome, studies have shown that MCC950 is a potential therapeutic drug for NLRP3 inflammasome-related syndromes (including autoinflammatory and autoimmune diseases). The studies mainly found that cells pre-treated with MCC950 inhibit the formation of ASC specks under pro-inflammatory conditions, and MCC950 can also reduce the production of IL-1β *in vivo* and attenuate the severity of disease models of multiple sclerosis, such as experimental autoimmune encephalomyelitis (EAE); in addition, in a CAPS mouse model, treatment with MCC950 can reduce the lethality of newborn mice and extend their lifespan by nearly 20 days. MCC950 also shows efficacy in inhibiting the release of IL-18 in plasma for Muckle Wells syndrome. In the drug development for rheumatoid arthritis, MCC950 had entered phase II clinical trials, but the clinical trials were suspended due to excessive hepatotoxicity. In addition to MCC950, CN112654350A discloses a series of NLRP3 antagonists for treating inflammatory or autoimmune disorders. However, NLRP3 antagonists are still under development and are combined with anti-TNF α agents, which clearly lack the advantages of drugs that directly target NLRP3 itself.

5-FUMCL is a derivative of a sesquiterpene compound, and its chemical structural formula is shown below:

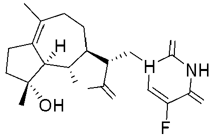

Currently, there have been no reports on the pharmacological research of 5-FUMCL as an anti-inflammatory drug that directly targets the NLRP3 inflammasome.

### Summary

### Technical Problem

An object of the present disclosure is to provide use of an inhibitor specifically targeting NLRP3 inflammasome.

### Technical Solution

To achieve the object described above, the following technical solutions are adopted in the present disclosure:
Provided is use of any one or more of 5-FUMCL and a pharmaceutically acceptable salt, a stereoisomer, and a precursor compound thereof for manufacturing an anti-inflammatory medicament that directly targets NLRP3 inflammasome.

As a preferred embodiment, the medicament is used for preventing or treating an inflammatory disease related to abnormal activation of the NLRP3 inflammasome.

As a preferred embodiment, the inflammatory disease related to the abnormal activation of the NLRP3 inflammasome is any one of hereditary NLRP3-dependent autoinflammatory diseases, metabolic diseases caused by NLRP3, diseases caused by crystal and protein aggregation, acute tissue injury, and chronic inflammation.

As a preferred embodiment, the inflammatory disease related to the abnormal activation of the NLRP3 inflammasome is any one of cryopyrin-associated periodic syndrome (CAPS), familial cold autoinflammatory syndrome (FCAS), Muckle-Wells syndrome (MWS), inflammatory bowel disease (IBD), type II diabetes, obesity, non-alcoholic steatohepatitis, aging, tumor, atherosclerosis, gout, renal dysfunction, Alzheimer's disease (AD), Parkinson syndrome, sepsis, endotoxemia, peritonitis, war wound, cardiovascular stress, myocardial hypertrophy, and the like.

As a preferred embodiment, the medicament may inhibit activation of the NLRP3 inflammasome and/or pyroptosis of cells (e.g., macrophages).

As a preferred embodiment, the medicament has one or more of the following anti-inflammatory effects:
(i) inhibiting LPS- and/or MSU-induced secretion of IL-1β, IL-6, and IL-18 in serum;
(ii) inhibiting LPS- and/or MSU-induced secretion of IL-1β, IL-6, and IL-18 in joints and/or peritoneal fluid;
(iii) inhibiting MSU-induced joint swelling;
(iv) inhibiting the number of neutrophils in the MSU-induced peritoneal fluid.

As a preferred embodiment, the medicament (e.g., a medicament comprising 5-FUMCL) may be used for preventing or treating acute systemic inflammation.

As a preferred embodiment, the medicament (e.g., a medicament comprising 5-FUMCL) may be used for preventing or treating acute peritonitis.

As a preferred embodiment, the medicament (e.g., a medicament comprising 5-FUMCL) may be used for preventing or treating gouty arthritis.

As a preferred embodiment, the medicament (e.g., a medicament comprising 5-FUMCL) may inhibit activation of the NLRP3 inflammasome *in vivo* and *in vitro.*

As a preferred embodiment, in addition to the active ingredient such as 5-FUMCL, the medicament further comprises a pharmaceutically acceptable carrier and/or other excipients that do not affect the efficacy of the active ingredient. For example, the medicament further comprises a sweetener for improving taste, an antioxidant for preventing oxidation, an excipient necessary for various formulations, and the like. As a preferred embodiment, the dosage form of the medicament is not limited, as long as it is a dosage form that enables the effective delivery of the active ingredient *in vivo,* such as a tablet, a capsule, a granule, a powder, a syrup, a solution, a suspension, an injection, a tincture, a sustained-release preparation, an oral liquid, an injection aerosol, a buccal tablet, an electuary, a pill, a dripping pill, a pulvis, an inhalation powder, and other common dosage forms, or a nano-formulation and other sustained-release dosage forms.

The "pharmaceutically acceptable salt" described above refers to a salt formed from 5-FUMCL and a pharmaceutically acceptable inorganic acid or organic acid, in which the inorganic acid is hydrochloric acid, hydrobromic acid, phosphoric acid, nitric acid, or sulfuric acid; the organic acid is formic acid, acetic acid, propionic acid, butanedioic acid, 1,5-naphthalenedisulfonic acid, asiatic acid, oxalic acid, tartaric acid, lactic acid, salicylic acid, benzoic acid, valeric acid, diethylacetic acid, malonic acid, succinic acid, fumaric acid, pimelic acid, adipic acid, maleic acid, malic acid, sulfamic acid, phenylpropionic acid, gluconic acid, ascorbic acid, nicotinic acid, isonicotinic acid, methanesulfonic acid, p-toluenesulfonic acid, citric acid, or an amino acid. The "pharmaceutically acceptable" means being suitable for use in humans without excessive adverse side effects (such as toxicity, irritation, and allergy), i.e., with a reasonable benefit/risk ratio. Pharmacological experiments are usually performed using a parent drug (e.g., 5-FUMCL), and in order to improve the solubility of the parent drug, the parent drug may usually be converted into a salt form in pharmaceutical practice.

The "stereoisomer" described above refers to an isomer resulting from different spatial arrangements of atoms in the 5-FUMCL molecule, e.g., a cis-trans isomer, a diastereoisomer, a conformational isomer, and the like.

### Beneficial Effects

Beneficial effects of the present disclosure are as follows:
Through pharmacological experiments, the present disclosure has revealed that 5-FUMCL can be used for manufacturing a medicament for the prevention and treatment of NLRP3 inflammasome-related diseases, that is, 5-FUMCL or a pharmaceutically acceptable salt, a stereoisomer, or the like thereof is used as an anti-inflammatory active pharmaceutical ingredient, which exerts a significant therapeutic effect on inflammation-related diseases such as acute systemic inflammation, acute peritonitis, and gouty arthritis by using its inflammation-blocking activity, and has no toxic and side effects. Meanwhile, the pharmacological experiments have also revealed that 5-FUMCL is an inhibitor specifically targeting NLRP3 inflammasome.

Furthermore, in the present disclosure, an acute inflammation model is constructed by intraperitoneal injection of LPS (lipopolysaccharide) after intragastric administration of 5-FUMCL, and MCC950 is used as a positive control. It has been found that 5-FUMCL can inhibit the secretion of IL-6 and IL-1β *in vivo.*

Furthermore, the present disclosure has also found that after intraperitoneal injection of 5-FUMCL, symptoms related to gouty arthritis induced by injection of MSU (sodium urate) into the knee joint cavity can be significantly ameliorated (the release of inflammatory cytokines at the joints and the joint swelling degree are reduced), while the corresponding anti-inflammatory effect is not shown in a gouty arthritis model in which the NLRP3 molecule is knocked out.

Furthermore, the present disclosure has also found that after intraperitoneal injection of 5-FUMCL, symptoms related to acute peritonitis induced by intraperitoneal injection of MSU can be significantly ameliorated (the release of inflammatory cytokines and the number of neutrophils in serum and abdominal cavity are reduced), while the corresponding anti-inflammatory effect is not shown in an acute peritonitis model in which the NLRP3 molecule is knocked out.

Furthermore, the above pharmacological experimental results for different inflammatory disease models show that 5-FUMCL can prevent and treat diseases related to abnormal activation of the NLRP3 inflammasome, such as a hereditary NLRP3-dependent autoinflammatory disease, a metabolic disease caused by NLRP3, a disease caused by crystal and protein aggregation, acute tissue injury, chronic inflammation, and the like, by specifically targeting NLRP3 inflammasome activation.

### BRIEF DESCRIPTION OF THE DRAWINGS

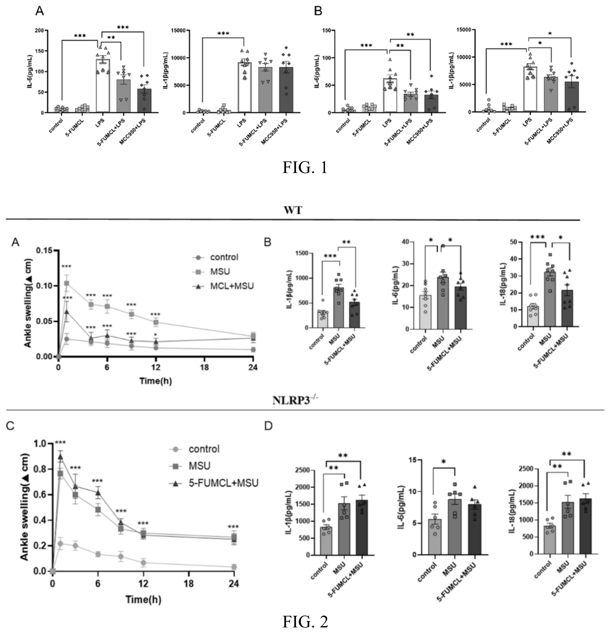

FIG. 1 shows the experimental results of ameliorating LPS-induced systemic inflammation by 5-FUMCL, in which A shows the content of IL-6 and IL-1β in the serum 7 h after LPS treatment, and B shows the content of IL-6 and IL-1β in the serum 12 h after LPS treatment; * P < 0.05, ** P < 0.01, and *** P < 0.001.
FIG. 2 shows the experimental results of ameliorating MSU-induced gouty arthritis by 5-FUMCL, in which A shows the change in knee joint width of WT mice 24 h after treatment, B shows the content of IL-1β, IL-6, and IL-18 in the joint culture supernatant of WT mice 24 h after treatment, C shows the change in knee joint width of NLRP3^{-/-} mice 24 h after treatment, and D shows the content of IL-1β, IL-6, and IL-18 in the joint culture supernatant of NLRP3^{-/-} mice 24 h after treatment; * P < 0.05, ** P < 0.01, and *** P < 0.001.

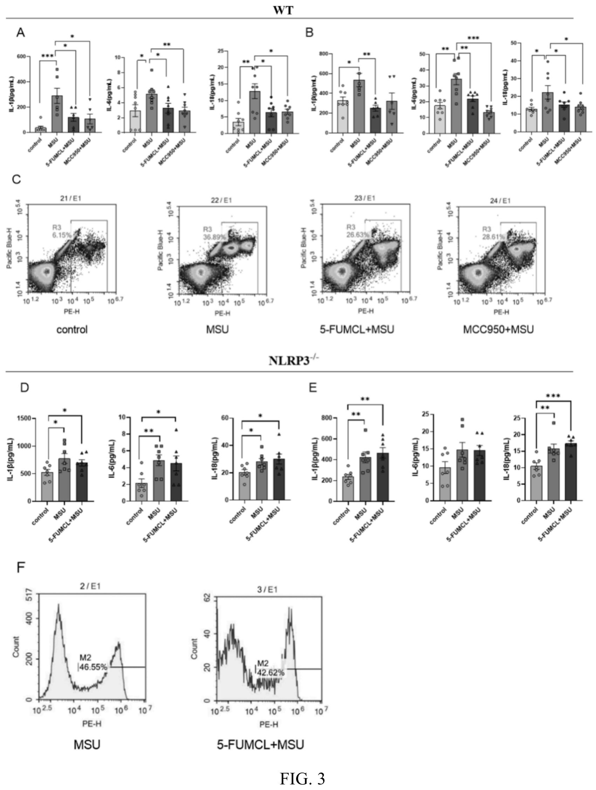

FIG. 3 shows the experimental results of ameliorating MSU-induced acute peritonitis by 5-FUMCL, in which A shows the content of IL-1 β, IL-6, and IL-18 in the serum of WT mice 6 h after intraperitoneal injection of MSU, B shows the content of IL-1β, IL-6, and IL-18 in the peritoneal fluid of WT mice 6 h after intraperitoneal injection of MSU, and C shows the proportion of neutrophils to the cells in the peritoneal fluid of WT mice 6 h after intraperitoneal injection of MSU, D shows the content of IL-1β, IL-6, and IL-18 in the serum of NLRP3^{-/-} mice 6 h after intraperitoneal injection of MSU, E shows the content of IL-1β, IL-6, and IL-18 in the peritoneal fluid of NLRP3^{-/-} mice 6 h after intraperitoneal injection of MSU, and F shows the proportion of neutrophils to the cells in the peritoneal fluid of NLRP3^{-/-} mice 6 h after intraperitoneal injection of MSU; * P < 0.05, ** P < 0.01, and *** P < 0.001.

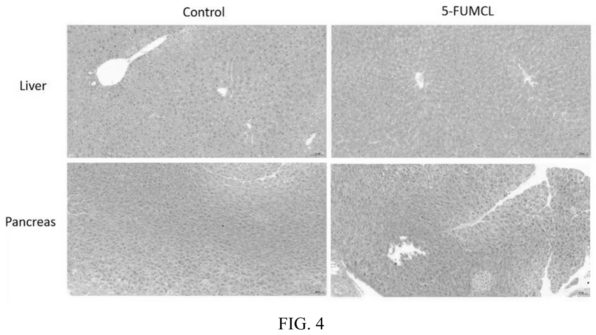

FIG. 4 shows the experimental results of toxic and side effects of 5-FUMCL.

### Best Embodiment of the Present Disclosure

The present disclosure will be further illustrated in detail with reference to the following accompanying drawings and examples. The examples are merely intended to illustrate the present disclosure rather than limit the protection scope of the present disclosure.

### (1) Experiment on amelioration of LPS-induced systemic inflammation by 5-FUMCL

### a1. Sample preparation

5-FUMCL was dissolved in dimethylsulfoxide (DMSO) to obtain a 40 mg/mL stock solution, and the stock solution was diluted with PBS (pH 7.4) to obtain a 0.4 mg/mL working solution before intragastric administration. The dose of 5-FUMCL for the *in vivo* experiment was 50 mg/kg. A stock solution and a working solution of MCC950 were prepared in PBS (pH 7.4) at a concentration of 4 mg/mL, and the dose of MCC950 for the *in vivo* experiment was 50 mg/kg.

### a2. Experimental method

C57BL/6J mice (n = 8 or 6 mice/group) aged 6-8 weeks and weighing about 20 g were selected. The mice in the experimental group were intragastrically administered 50 mg/kg 5-FUMCL, and were intraperitoneally injected with 10 mg/kg LPS 30 min later (the group was designated as 5-FUMCL + LPS). The mice in the blank control group were intragastrically administered only an equal volume of PBS (the group was designated as control); the mice in the negative control group were intragastrically administered only 5-FUMCL (the group was designated as 5-FUMCL); the mice in the model group were intraperitoneally injected only with 10 mg/kg LPS (i.e., the LPS group); and the mice in the positive drug group were intragastrically administered 50 mg/kg MCC950, and were intraperitoneally injected with 10 mg/kg LPS 30 min later (the group was designated as MCC950 + LPS).

### a3. Determination of inflammatory cytokines (the determination was performed with reference to the instructions in kits) and statistical method (the comparison among experimental data of the groups was performed using one-way ANOVA statistical analysis)

### a4. Experimental results

As shown in FIG. 1, the results show that 5-FUMCL was capable of reducing the content of IL-6 and IL-1β in the serum, i.e., 5-FUMCL has a protective effect on LPS-induced acute inflammation model mice.

### (2) Experiment on amelioration of MSU-induced gouty arthritis by 5-FUMCL

### b1. Sample preparation

5-FUMCL was dissolved in dimethylsulfoxide (DMSO) to obtain a 40 mg/mL stock solution, and the stock solution was diluted with PBS (pH 7.4) to obtain a 0.4 mg/mL working solution before injection. The dose of 5-FUMCL for the *in vivo* experiment was 50 mg/kg.

### b2. Experimental method

C57BL/6J mice, i.e., WT mice (n = 8 or 6 mice/group), aged 6-8 weeks and weighing about 20 g were selected. The mice in the experimental group were intraperitoneally administered 50 mg/kg 5-FUMCL, and after 30 min, MSU was injected into the knee joint at 0.5 mg/mouse. 24 h after the injection of MSU, the knee joint was separated and cultured in an OPTI-MEM culture medium for 1 h, and then the culture medium was centrifuged to obtain a joint culture supernatant (the group was designated as 5-FUMCL + MSU). The mice in the blank control group were intraperitoneally injected only with an equal volume of PBS (the group was designated as control). For the mice in the model group, only MSU was injected into the knee joint at 0.5 mg/mouse (i.e., the MSU group).

NLRP3 KO mice (Shanghai Model Organisms Center, Inc.; heterozygous mice in which the Nlrp3 gene was knocked out were obtained using CRISPR/Cas9 technology, and homozygous mice in which NLRP3 was knocked out were obtained by mating), i.e., NLRP3^{-/-} mice (n = 6 mice/group), aged 6-8 weeks and weighing about 20 g were selected. The mice in the experimental group were intraperitoneally administered 50 mg/kg 5-FUMCL, and after 30 min, MSU was injected into the knee joint at 0.5 mg/mouse. 24 h after the injection of MSU, the knee joint was separated and cultured in an OPTI-MEM culture medium for 1 h, and then the culture medium was centrifuged to obtain a joint culture supernatant (the group was designated as 5-FUMCL + MSU). The mice in the blank control group were intraperitoneally injected only with an equal volume of PBS (the group was designated as control). For the mice in the model group, only MSU was injected into the knee joint at 0.5 mg/mouse (i.e., the MSU group).

### b3. Evaluation of knee joint swelling degree, determination of inflammatory cytokines, and statistical method

In the evaluation of the knee joint swelling degree, the maximum width of the knee joint was measured using a vernier caliper; the determination of inflammatory cytokines was performed with reference to the instructions in kits; and the comparison among experimental data of the groups was performed using one-way ANOVA statistical analysis.

### b4. Experimental results

As shown in FIG. 2, the results show that 5-FUMCL was capable of ameliorating MSU-induced arthritic responses in WT mice, and the content of IL-1β, IL-6, and IL-18 in the joint culture supernatant was significantly reduced; however, under the same conditions, the above protective effect of 5-FUMCL on NLRP3^{-/-} mice disappeared. The above results indicate that 5-FUMCL has a significant anti-inflammatory effect on MSU-induced gouty arthritis, and the anti-inflammatory effect acts through the NLRP3 inflammasome.

### (3) Experiment on amelioration of MSU-induced acute peritonitis by 5-FUMCL

### c1. Sample preparation

5-FUMCL was dissolved in dimethylsulfoxide (DMSO) to obtain a 40 mg/mL stock solution, and the stock solution was diluted with PBS (pH 7.4) to obtain a 0.4 mg/mL working solution before injection. The dose of 5-FUMCL for the *in vivo* experiment was 50 mg/kg. A stock solution and a working solution of MCC950 were prepared in PBS (pH 7.4) at a concentration of 4 mg/mL, and the dose of MCC950 for the *in vivo* experiment was 50 mg/kg.

### c2. Experimental method

C57BL/6J mice, i.e., WT mice (n = 8 or 6 mice/group), aged 6-8 weeks and weighing about 20 g were selected. The mice in the experimental group were intraperitoneally administered 50 mg/kg 5-FUMCL, and were intraperitoneally injected with 0.5 mg/mouse MSU 30 min later (the group was designated as 5-FUMCL + MSU). The mice in the blank control group were intraperitoneally injected only with an equal volume of PBS (the group was designated as control). The mice in the model group were intraperitoneally injected only with 0.5 mg/mouse MSU (i.e., the MSU group). The mice in the positive drug group were intraperitoneally administered 50 mg/kg MCC950, and were intraperitoneally injected with 0.5 mg/mouse MSU 30 min later (the group was designated as MCC950 + MSU).

NLRP3 KO mice (Shanghai Model Organisms Center, Inc.; heterozygous mice in which the Nlrp3 gene was knocked out were obtained using CRISPR/Cas9 technology, and homozygous mice in which NLRP3 was knocked out were obtained by mating), i.e., NLRP3^{-/-} mice (n = 6 mice/group), aged 6-8 weeks and weighing about 20 g were selected. The mice in the experimental group were intraperitoneally administered 50 mg/kg 5-FUMCL, and were intraperitoneally injected with 0.5 mg/mouse MSU 30 min later (the group was designated as 5-FUMCL + MSU). The mice in the blank control group were intraperitoneally injected only with an equal volume of PBS (the group was designated as control). The mice in the model group were intraperitoneally injected only with 0.5 mg/mouse MSU (i.e., the MSU group).

### c3. Determination of neutrophils and inflammatory cytokines, and statistical method

Neutrophils were detected using a flow cytometer; the determination of inflammatory cytokines was performed with reference to the instructions in kits; and the comparison among experimental data of the groups was performed using one-way ANOVA statistical analysis.

### c4. Experimental results

As shown in FIG. 3, the results show that 5-FUMCL was capable of significantly reducing the content of IL-1β, IL-6, and IL-18 in the serum and peritoneal fluid of WT mice after MSU induction, and the proportion of neutrophils to the cells in the peritoneal fluid was significantly reduced; however, under the same conditions, the above protective effect of 5-FUMCL on NLRP3^{-/-} mice after MSU induction disappeared. The above results indicate that 5-FUMCL has a significant anti-inflammatory effect on MSU-induced acute peritonitis, and the anti-inflammatory effect acts through the NLRP3 inflammasome.

### (4) Toxic and side effects

The mice were intraperitoneally injected with 50 mg/kg 5-FUMCL daily for 120 consecutive days. Liver and pancreas were collected for HE staining. No significant inflammatory cell infiltration and injury was observed (FIG. 4).

### (5) Preparation of 5-FUMCL inhalation powder

20 g of 5-FUMCL was mixed with 180 g of an excipient (in a mass ratio of maltodextrin:lactose of 7:3), and then completely dissolved in 50% ethanol. The resulting solution was spray-dried to obtain an inhalation powder. The inhalation powder was precisely weighed out and filled into No. 3 capsules, with a sample size of 20 mg per capsule, where the content of 5-FUMCL was 2 mg. The inhalation powder features good stability, is easy to carry, is convenient for patients to use, has high drug concentration in the lung, reduces systemic drug exposure, and can effectively improve the therapeutic effect on severe pneumonia.

### (6) Preparation of 5-FUMCL tablet

10 g of 5-FUMCL was mixed with 87.5 g of an excipient (in a mass ratio of white dextrin:lactose of 7:3), and then 95% ethanol was added. The resulting mixture was granulated, dried, and sized (sieved). 2.5 g of sodium stearate was added. The mixture was mixed homogenously and tableted according to a tablet weight of 100 mg, where the content of 5-FUMCL was 10 mg.

### (7) Preparation of 5-FUMCL capsule

15 g of 5-FUMCL was mixed with 135 g of an excipient (in a mass ratio of white dextrin:lactose of 7:3), and then 95% ethanol was added. The resulting mixture was granulated, dried, sized (sieved), and filled into capsules, each capsule weighing 150 mg, where the content of 5-FUMCL was 15 mg.

### (8) Preparation of 5-FUMCL powder for injection

1 g of 5-FUMCL and 5 g of mannitol were dissolved in 170 mL of water for injection, and the solution was mixed homogenously for the first time and then brought to 200 mL. The resulting solution was filtered, and the filtrate was filled into vials at 1 mL per vial, lyophilized, sealed, and sterilized to obtain a powder for injection containing 5 mg of 5-FUMCL in each vial.

### (9) Dosage and administration

Capsule: Adults take 2-4 capsules at a time, 3 times a day; children under 12 years of age take half the adult dose.

Tablet: Adults take 3-6 tablets at a time, 3 times a day; children under 12 years of age take half the adult dose.

Powder for injection: Adults take 1 vial at a time by intravenous bolus injection, intravenous drip, or intramuscular injection, 3 times a day; children under 12 years of age take half the adult dose.

### Industrial Applicability

In conclusion, the present disclosure reveals experimentally that 5-FUMCL has significant anti-inflammatory effects on LPS-induced acute systemic inflammation, as well as MSU-induced gouty arthritis and acute peritonitis; moreover, 5-FUMCL can specifically target the NLRP3 inflammasome. These experimental results show that 5-FUMCL can inhibit the secretion of inflammatory cytokines IL-1β, IL-6, and IL-18 by inhibiting the abnormal activation of the NLRP3 inflammasome, thereby exhibiting significant clinical medicinal value in the prevention and treatment of NLRP3 inflammasome-related inflammatory diseases. 5-FUMCL can particularly be used as an active ingredient for manufacturing an anti-inflammatory medicament for the treatment of inflammation-related diseases such as systemic inflammation, gouty arthritis, peritonitis, and the like.

## Claims

1. Use of any one or more of 5-FUMCL or a pharmaceutically acceptable salt, a stereoisomer, and a precursor compound of 5-FUMCL for manufacturing an anti-inflammatory medicament that targets NLRP3 inflammasome.

2. A compound for use in reducing inflammation by targeting NLRP3 inflammasome, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

3. Use of a compound in reducing inflammation by targeting NLRP3 inflammasome, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

4. The use according to claim 1, wherein the medicament is used for preventing or treating an inflammatory disease related to abnormal activation of the NLRP3 inflammasome.

5. A compound for use in preventing or treating an inflammatory disease related to abnormal activation of NLRP3 inflammasome, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

6. Use of a compound in preventing or treating an inflammatory disease related to abnormal activation of NLRP3 inflammasome, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

7. The use according to claim 4, 5, or 6, wherein the inflammatory disease related to the abnormal activation of the NLRP3 inflammasome is any one of a hereditary NLRP3-dependent autoinflammatory disease, a metabolic disease caused by NLRP3, a disease caused by crystal and protein aggregation, acute tissue injury, and chronic inflammation.

8. The use according to claim 4, 5, or 6, wherein the inflammatory disease related to the abnormal activation of the NLRP3 inflammasome is cryopyrin-associated periodic syndrome, familial cold autoinflammatory syndrome, Muckle-Wells syndrome, inflammatory bowel disease, type II diabetes, obesity, non-alcoholic steatohepatitis, aging, a tumor, atherosclerosis, gout, renal dysfunction, Alzheimer's disease, Parkinson syndrome, sepsis, endotoxemia, peritonitis, war wound, cardiovascular stress, or myocardial hypertrophy.

9. The use according to claim 1, wherein the medicament inhibits activation of the NLRP3 inflammasome and/or pyroptosis.

10. The use according to claim 1, wherein the medicament has one or more of the following anti-inflammatory effects:
(i) inhibiting secretion of IL-1β, IL-6, and IL-18 in serum;
(ii) inhibiting secretion of IL-1β, IL-6, and IL-18 in joints and/or peritoneal fluid;
(iii) inhibiting joint swelling; and
(iv) inhibiting the number of neutrophils in peritoneal fluid.

11. Use of any one or more of 5-FUMCL or a pharmaceutically acceptable salt, a stereoisomer, and a precursor compound of 5-FUMCL for manufacturing a medicament for the prevention or treatment of acute systemic inflammation.

12. A compound for use in preventing or treating acute systemic inflammation, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

13. Use of a compound in preventing or treating acute systemic inflammation, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

14. Use of any one or more of 5-FUMCL or a pharmaceutically acceptable salt, a stereoisomer, and a precursor compound of 5-FUMCL for manufacturing a medicament for the prevention or treatment of acute peritonitis.

15. A compound for use in preventing or treating acute peritonitis, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

16. Use of a compound in preventing or treating acute peritonitis, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

17. Use of any one or more of 5-FUMCL or a pharmaceutically acceptable salt, a stereoisomer, and a precursor compound of 5-FUMCL for manufacturing a medicament for the prevention or treatment of gouty arthritis.

18. A compound for use in preventing or treating gouty arthritis, wherein the compound is:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

19. Use of a compound in preventing or treating gouty arthritis:
(i) 5-FUMCL;
(ii) any one of a pharmaceutically acceptable salt, a stereoisomer, and a precursor compound of 5-FUMCL;
(iii) or any combination of two or more of the compounds in (i) and (ii) above.

20. Use of any one or more of 5-FUMCL or a pharmaceutically acceptable salt, a stereoisomer, and a precursor compound of 5-FUMCL for inhibiting activation of NLRP3 inflammasome.
